# EUROPEAN PATENT APPLICATION

(11) **EP 3 098 278 A1**
(43) Date of publication of application: **30.11.2016**
(21) Application number: 16172288.9
(22) Date of filing: 03.05.2015
(51) Int. Cl.: C09J 7/02

(54) **ROLL OF ADHESIVE TAPE FOR PACKAGING**

(30) Priority: 02.05.2014 IT BO20140252
(62) Divisional of application: 15166156.8
(71) Applicant: MAGIS S.P.A., 50050 Cerreto Guidi (FI) (IT)
(72) Inventor: MARZI, Marco, 50050 Cerreto Guidi (FI) (IT); MANNUCCI, Vanni, 50050 Cerreto Guidi (FI) (IT)
(74) Representative: Mincone, Antimo

(57) **Abstract**

A roll of self-adhesive tape for packaging and similar uses of the type formed by an inner core on which is wound a tape in plastic material rendered adhesive by means of association of an adhesive, characterized in that said tape (3) is composed of a non-textile film (3) in polyethylene terephthalate or PET having a thickness between 8 and 15 microns (micrometers) and an adhesive layer in hot-melt glue having a basis weight of between 8 and 22 grams per square meter.

## Description

The present invention relates to a self-adhesive tape for packaging, sealing boxes, and similar uses.

In their usual conformation, the adhesive tapes for packaging are made by wrapping, around a cardboard core, a tape of plastic material, usually polypropylene, having a side coated by an adhesive that must have an adhesive strength such as to permit the unwrapping of the roll (which happens in many cases also thanks to the presence of a release agent on the side opposite to the adhesived side) and the subsequent fastening action on the materials to be bonded with the tape.

EP-0599789B2 was been granted for a roll of self-adhesive tape for packaging formed by a tape in plastic material wound on a tubular core made of cardboard, in which the diameter of the cardboard core is of the order of 38,2mm, in which the length of the tape is between approximately 105m and 220m and the outer diameter of the roll is about 120mm.

The adhesive tape of EP-0599789B2, as the other self-adhesive tapes currently on the market, has some drawbacks which are brilliantly solved by the present invention, as will be better clarified in the following of the present description, in which will also be more evident the many advantages that characterize the self-adhesive tape of the invention.

Among the advantages of the invention can be mentioned the following:
- with the present invention it is possible to provide a self-adhesive tape which, at the same volume, or at the same roll diameter, extends for linear values greater than the adhesive tapes of the traditional type, with obvious advantages both for the end user (greater autonomy of use of the single roll) and for logistics (increased amount of square meters with equal volume);
- the tape in question has a greater mechanical resistance (for example to tensile stresses) compared to a conventional adhesive tape of the same weight per square meter of the support;
- the possibility to use a substrate structure having lower weights per square meter which creates an improved environmental impact, since with the tape of the present invention the waste materials are reduced (in terms of weight), and an optimization of the production processes is obtained, since can be actually used jumbo rolls longer, thereby reducing the productive downtime;
- the adhesive tape of the invention adheres better to the pack or the box to be closed, because, thanks to the reduced thickness of the substrate, it can copy in a better way the profile and the surface shape of the object to be coated; in this way is required, therefore, a smaller amount of glue for the same adhesive efficacy;
- in the case of hot-melt glues there is a better anchorage of the release agent (release), necessary with this type of adhesive masses in order to allow it to unwind.

This result is achieved, according to the present invention, by adopting the idea of making an adhesive tape for packaging having the characteristics indicated in claim 1. Other features of the present invention are described in the dependent claims.

The advantages of the present invention will be more evident thanks to the description which follows, made with reference to the attached figures, to be understood in the sense of non limiting examples, in which:
- Fig. 1 is a schematic perspective view of a roll of adhesive tape made in accordance with the invention;
- Fig. 2 is a side view of the example of Fig.1;
- Fig. 3 is an enlarged side view of a possible embodiment of a tape in accordance with the invention;
- Fig. 4 schematically shows a possible example of the production stages of an adhesive tape according to the invention.

The present invention relates to a roll of adhesive tape (marked with 1 in the drawings) that can be used for packaging, or to join together boxes, containers, their parts, etc .. The roll (1) is substantially composed of a central core (2) and a tape (3) wound around the same core (2) to form the body or the useful part (33) of the roll.

The central core (2) can be realized in cardboard and can have a diameter (D2) variable between 20 and 160 mm.

The tape (3) is formed by a film (30) on which is made to adhere, for example coated, a layer of adhesive (31) that provides the adhesive power to tape. The width (L3) is variable depending on the intended use of the adhesive tape (3).

Advantageously, in accordance with the invention, the film that constitutes the support structure (30) of the tape it is polyethylene terephthalate or PET.

In particular it is a film (non textile) PET with a thickness (H30) comprised between 8 and 23 µm.

In a particularly effective embodiment the thickness (H30) can be comprised between 8 and 15 µm.

The layer of glue has a grammage comprised between 8 and 32 grams per square meter, with an embodiment particularly effective between 8 and 22 grams per square meter.

The diameter (D1) of the roll (1) can be variable and will be determined by the total diameter of the tape, by the diameter (D2) of the specific central core, and by the thickness of the chosen PET base as well as from that of the applied adhesive mass.

In the production procedure of the present invention, it is possible to use coils of material (PET film) having a length very high; this results in increased productivity by reduction in downtime or slowdowns caused by the change of the coil; furthermore, the need for a smaller number of coils determines a lower contribution of waste.

On the film that defines the base (30) of the belt (3) can be used different types of adhesive (31) to determine the adhesive power as for example acrylic glue or hot-melt (also called HM in the present description).

When the film (30) is coated with an adhesive (31) of hot-melt type, a greater anchoring power of the release agent is obtained thanks to the higher polarity of the PET support, which, compared to other traditional supports, allows a greater anchoring of the release agent due to the interaction forces of various kinds, such as hydrogen bonds, Van Der Waals forces, etc..

In addition, the tape (3) can advantageously be printed using the so-called sandwich technique, i.e. with protected printing; in practice, the print is performed on one side (34) of the film (30) PET and subsequently on the same side the adhesive is applied. In this way, the printing is located between two layers firmly united to each other: the film (30) on one side and the glue (31) on the other.

From what expressed, the advantages of the present invention are evident because a tape with a support made with PET film shows several advantages compared to one that has a BOPP support.

Experimental tests performed by the Applicant have shown, inter alia, the following advantages:
- the breaking loads for a tape with PET film are similar to those with a BOPP substrate having a thicknesses 50% greater: in practice, a 15 µm PET film has a break load value similar to a BOPP film of 23 µm; a 12 µm PET film similar to that of 19 µm in BOPP, and a 23 µm has a value similar to a 35 µm BOPP;
- PET adhesive tapes have greater flatness and dimensional stability;
- PET adhesive tapes have higher temperature resistance, especially at high temperatures.

All this allows to utilize supports thinner, with many advantages:
- greater conformability to the support where the product is applied, thus facilitating the adhesion even in cases of imperfect pressure applied;
- greater autonomy in terms of linear meters, with the same diameter of the roll: this feature allows to maximize the productivity, reducing the replacements on the machine, and optimization the transport (less volume for the same square meters and cores);
- optimal easy cutting, being intermediate between that of a PVC tape (which is greatly deformed during the cut) and that of a tape BOPP with easy tear (which, instead, tears too easily).

Compared to PVC substrates additional advantages are to be found also in the environmental sustainability of the product: in fact the PVC tapes have production processes that cannot be defined as eco-friendly (both as regards the support and with regard to the adhesive mass). The tapes PET instead, having both acrylic and hot melt adhesive masses, show a much more limited environmental impact.

Particularly advantageous are the relations between the roll diameter and the length of the adhesive tape. In particular, for PET tapes with hot-melt glue neutral (not printed), it is advantageously usable as support a film of 12 µm; in this case the sleeve or core (2) it may be only of 50.8 mm (2 inches), so as to obtain a very good compactness / autonomy ratio; also with a PET film of 15 µm excellent compactness / autonomy ratio can be obtained using a core of 50.8 mm or 76.2 mm (2 inches or 3 inches). The weights of glue can be similar to those of the products in BOPP; consequently, also the performance of the adhesive mass tested in laboratory (tests of peel and tack for example), will be similar; the benefits of the thin support will make however greater the adhesive efficiency in practice, as specified above.

For tapes with printed hot melt adhesive it was particularly advantageous to use a support to be 15 µm with core of 50.8 mm or 76.2 mm (2 or 3 inches). The weights of glue are similar to those of the products in BOPP; consequently, also the performance of the adhesive mass tested in the laboratory (tests of peel and tack for example), will be similar; the benefits of the thin support will make however in practical terms greater the adhesive efficiency, as specified above.

Another great advantage derives, for the printable type, by the fact that the tape not requires the corona treatment before printing, thanks to the intrinsic polarity of the substrate. We have to consider that the corona treatment in the printing stage from above is always a delicate stage and not desirable: in fact a too high treatment could lead to defects in the unwinding (with possible loss of glue on the back or a break in the unwinding), while a too low treatment could result in a non perfect anchorage of the printing (with possible set-off). Moreover, the corona treatment requires energy costs and submit the operator at the exposure to ozone (subproduct of corona treatment with air plasma).

The measurable benefits of this invention are related, mainly, to the mechanical strength: PET has a breaking load which is 50% greater than the equivalent thickness of BOPP (according to methodology AFERA 5004). In practice, a tape with PET film of 23 µm offers a strength similar to those of a tape with BOPP support of 35 µm. And a tape with a PET 15 µm support is similar to a 23 µm in BOPP.

Regarding to the adhesiveness (peel test 90° on steel, according to methodology AFERA 5001) a greater adhesion can also be observed (increase of about 20%); this appears mainly due to the reduced thickness which slightly changes the angle of attack (which nominally would be 90° but is influenced by the thickness: a higher thickness reduces the effective angle in the release area) and facilitates the adhesion in the reduced time required by the test. From this result it can be concluded that the application on cardboard (carton sealing - closing boxes) will be better thanks to the reduced thickness that facilitates the adhesion.

Thanks to the present invention it is possible to provide rolls of tape having lengths extremely high in relation to the total diameter (D1) of the roll (1).

From the data of Table 1 are clear the advantages in terms of size of the rolls of the examples used. The first line refers to a tape with PET support of from 15 µm with 17 gsm of HM glue and core of 76.2 mm (3 inches); the second line refers to an equivalent tape with BOPP support of 23 µm with 17 gsm of HM glue and core of 76.2 mm (3 inches); the third line refers to a tape with PET support of 23 µm with 21 gsm of acrylic glue and core of 76.2 mm (3 inches); the fourth line relates to an equivalent tape with BOPP support of 35 µm with 21 gsm of acrylic glue and core of 76.2 mm (3 inches). The difference in length is clear.

**Table 1**

| Lenght | Thickness | Core(D2) | Gramm | Diam(D1) |
|---|---|---|---|---|
| (meters) | (µm) | (mm) | (g/mq) | (mm) |
| 90,0 | 15,0 | 76,2 | 17,0 | 97,3 |
| 72,0 | 23,0 | 76,2 | 17,0 | 97,3 |
| 90,0 | 23,0 | 76,2 | 21,0 | 104,1 |
| 70,8 | 35,0 | 76,2 | 21,0 | 104,2 |

In Table 2 it 1 is shown how it 1 is advantageous the tape (1) of the invention also using a core (2) of 25.4 mm (1 inch).

**Table 2**

| Lenght | Thickness | Core(D2) | Gramm | Diam(D1) |
|---|---|---|---|---|
| (meters) | (µm) | (mm) | (g/mq) | (mm) |
| 90,0 | 15,0 | 25,4 | 17,0 | 65,6 |
| 72,0 | 23,0 | 25,4 | 17,0 | 65,6 |
| 90,0 | 23,0 | 25,4 | 21,0 | 75,4 |
| 70,8 | 35,0 | 25,4 | 21,0 | 75,4 |

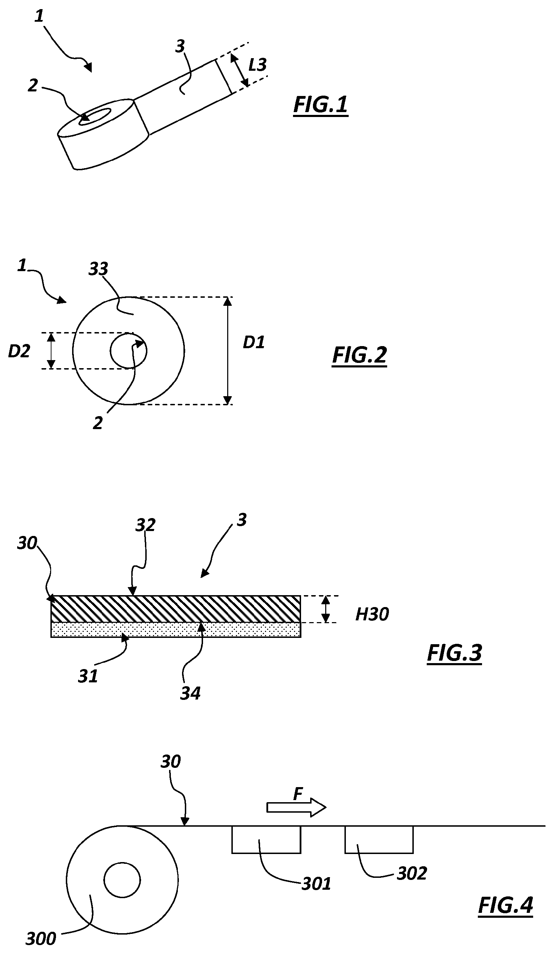

In Fig. 4 is schematically shown how to realize a tape in accordance with the invention. According to the invention, as specified in the independent claim of the patent description, the support of the tape is a PET film. The application of the tape is the packaging field (closing boxes and the like).

The process for the production of the roll of adhesive tape comprises the following steps: first unwinding from a reel (300) a Polyethylene terephthalate or PET film (non-textile). In Fig. 4 the arrow (F) indicates the direction of unwinding of the film. Thereafter, depending on the type of glue used, the process provides to apply a primer (301) in the case of acrylic glue or directly the glue in the case of hot-melt glue.

As described and illustrated has the value of an example of any modification or variant which falls within the protection defined by the claims.

## Claims

1. Roll of adhesive tape for packaging, of the type consisting of an inner core on which is wound a plastic tape which is made adhesive by means of the association of an adhesive, **characterized in that** said tape (3) is composed of a film (30) in Polyethylene terephthalate or PET having a thickness comprised between 8 and 15 µm and an adhesive layer in hot-melt glue having a basis weight of between 8 and 22 grams per square meter.

2. Roll of tape according to claim 1 **characterized in that** the tape (3 ) is printed on the upper or external face (32) without treatment.

3. Roll of tape according to claim 1 **characterized in that** the tape (3) is printed by means of sandwich technique, i.e. with protected printing: the printing being carried out on one side (34) of the structure in PET (30) and subsequently on the same side is applied the adhesive, so that the printing is located between two layers firmly joined together: the base structure (30) on one side and the glue (31) on the other.

4. Roll of tape according to one of the preceding claims **characterized in that** the core (2) has a diameter (D2) of 76,2 mm and the roll (1) has a length of 90 m and an external diameter of 97,3mm.

5. Roll of tape according to one of the preceding claims 1 to 3, **characterized in that** the core (2) has a diameter (D2) of 25,4 mm and the roll (1) has a length of 90 m and an external diameter of 65,6mm.

6. Process for the production of a roll of adhesive tape, **characterized in that** it comprises the following stages:
a) unwinding a polyethylene terephthalate or PET film from a reel (300);
b) application of glue of hot-melt type (302).
